## Europäisches Patentamt

## European Patent Office

## Office européen des brevets

(11) Veröffentlichungsnummer: **0 243 650**
**B1**

# EUROPÄISCHE PATENTSCHRIFT

(45) Veröffentlichungstag der Patentschrift:
06.06.90

(51) Int. Cl.⁵: **A61B 17/22**

(21) Anmeldenummer: 87104008.5

(22) Anmeldetag: 18.03.87

(54) Stosswellenquelle mit verbesserter Fokuszone.

(30) Priorität: 01.04.86 DE 3610850

(43) Veröffentlichungstag der Anmeldung:
04.11.87 Patentblatt 87/45

(45) Bekanntmachung des Hinweises auf die Patenterteilung:
06.06.90 Patentblatt 90/23

(84) Benannte Vertragsstaaten:
DE FR GB NL

(56) Entgegenhaltungen:
EP-A- 0 133 665
EP-A- 0 183 236
GB-A- 534 448
JP-A-58 085 694

JOURNAL OF THE ACOUSTICAL SOCIETY OF
AMERICA, Band 79, Nr. 2, Februar 1986, Seiten 566-570,
Acoustical Society of America, Woodbury, New York,
US; C.M. SEHGAL et al.: "Ultrasonic nonlinear
parameters and sound speed of alcohol-water
mixtures"

(73) Patentinhaber: Siemens Aktiengesellschaft,
Wittelsbacherplatz 2, D-8000 München 2(DE)

(72) Erfinder: Naser, Georg, Dipl.-Ing. (FH), Karlstrasse 10,
D-8502 Zirndorf(DE)
Erfinder: Reichenberger, Helmut, Dr.,
Begonienstrasse 28, D-8501 Eckental(DE)
Erfinder: Schwark, Hubert, Heinrich-Hertz-Strasse 15,
D-8520 Erlangen(DE)

## Beschreibung

Die Erfindung betrifft eine Stoßwellenquelle, insbesondere für einen Lithotripter, mit einer Druckquelle zur Erzeugung eines Druckwellenimpulses und mit einer im Ausbreitungsweg des Druckimpulses und im Abstand von der Druckquelle angeordneten Fokussierungseinrichtung zur Fokussierung des Druckwellenimpulses.

Eine Stoßwellenquelle der eingangs genannten Art für Zerstörung von Konkrementen im menschlichen Körper ist beispielsweise aus der DE-OS 3 328 051 bekannt. Dort ist als Druckquelle ein Stoßwellenrohr vorgesehen, welches eine Spule und, durch eine Isolierfolie getrennt, eine vorgelagerte Metallmembran umfaßt. Bei Anlegen eines Spannungssprungs an die Spule wird die Metallmembran abgestoßen; sie erzeugt in dem an ihr angrenzenden Medium einen Druckwellenimpuls. Dieser Druckwellenimpuls erreicht nach Durchqueren einer Vorlaufstrecke (Wasser) eine Linse, die als Fokussierungseinrichtung dient. Im geometrischen Fokus der Linse ist das zu zerstörende Konkrement plaziert.

Am Ort der Linse muß der Druckwellenimpuls vorgegebene Eigenschaften haben, um im Fokus als sogenannte Stoßwelle das Konkrement angreifen zu können. Es wurde nun erkannt, daß als maßgebliche Eigenschaft der Quotient aus Amplitude und Anstiegszeit des Druckwellenimpulses angesehen werden kann: Ist dieser Quotient am Ort der Linse nicht hinreichend groß, so kommt es nicht zur Ausbildung einer Stoßwelle. Um aber einen ausreichend großen Quotienten zu erzielen, ist es notwendig, daß sich der Druckwellenimpuls auf der sogenannten Vorlaufstrecke aufsteilt.

Die Vorlaufstrecke befindet sich, wie erwähnt, zwischen der Druckquelle und der Fokussierungseinrichtung. Bei gängigen Anordnungen dieser Art beträgt die Länge der Vorlaufstrecke ca. 20 cm.

Es hat sich nun im Experiment gezeigt, daß die radiale Druckverteilung im Verlauf der Vorlaufstrecke zunehmend einen stärker nach außen abfallenden Druck aufweist. Ist nahe an der Druckquelle die Druckverteilung über den größten Teil des Querschnittes noch nahezu konstant, so ist dies vor der Linse oder Fokussierungseinrichtung nicht mehr der Fall. Der Druck hat im mittleren Bereich einen maximalen Wert und fällt zum Rand hin unerwünscht stark ab; eine gleichmäßige Linsenaperturbelegung durch den Druckwellenimpuls wäre aber wünschenswert. Das hat zur Folge, daß die bei der Fokussierung effektiv wirksame Apertur deutlich kleiner ist als der Durchmesser der Linse. Der effektive Öffnungswinkel der Linse ist kleiner als eigentlich geometrisch möglich ist. Das hat nach den genannten Experimenten zur Folge, daß die Fokuszone, die z.B. durch die -6dB-Isobare, bezogen auf den Maximalwert des Druckes im Fokus, definiert ist, ein Längen-Breiten-Verhältnis von ca. 8 aufweist. Diese Effekte treten ebenso bei anderen Fokussierungseinrichtungen, wie z.B. einem Reflektor, auf.

Aus der prioritätsälteren, jedoch nicht vorveröffentlichten EP-A 0 183 236 ist eine Stoßwellenquelle bekannt, die als Druckquelle eine kugelkalottenartig ausgebildete, stoßartig antreibbare Metallmembran aufweist, deren nach innen gewölbte Fläche in Kontakt mit einem Übertragungsmedium steht, in das mittels der Membran fokussierte Druckwellenimpulse eingeleitet werden. Als Übertragungsmedium ist ein Medium mit stark nichtlinearem Kompressionsverhalten, dies entspricht einem hohen B/A-Wert, vorgesehen, wobei die Nichtlinearität bzw. der B/A-Wert größer als bei Wasser ist. Hierdurch wird der zur Ausbildung einer Stoßwelle erforderliche Laufweg verkürzt.

Aufgabe der Erfindung ist es, einen Lithotripter der eingangs genannten Art so auszugestalten, daß das Verhältnis von Länge zu Breite der Fokuszone verringert wird.

Diese Aufgabe wird erfindungsgemäß dadurch gelöst, daß sich zwischen der Druckquelle und der Fokussierungseinrichtung ein Stoff befindet, dessen B/A-Wert größer als der von Wasser und dessen akustische Impedanz kleiner oder gleich der von Wasser ist. Die stoffspezifische Größe B/A beschreibt für die Ausbreitung akustischer Wellen in einem flüssigen oder gasförmigen Medium die Abweichungen vom linearen Verhalten. Für Wasser hat B/A bei 20°C den Wert 5.

Mit dem Einbringen eines Stoffes mit hohem B/A-Wert in die Vorlaufstrecke zwischen Druckquelle und Fokussierungseinrichtung macht man sich den an sich bekannten physikalischen Effekt zunutze, daß eine Druckwelle sich schneller aufsteilt, wenn sie sich in einem Medium mit hohem B/A-Wert fortpflanzt. Steilt sich der Druckwellenimpuls schneller auf, so läßt sich die Länge der Vorlaufstrecke relativ kurz halten. Beim Einsatz von Rizinusöl mit einem B/A-Wert von etwa 10 als Stoff, der den beiden genannten Bedingungen genügt, ist eine Verkürzung der Strecke um ca. 40% im Vergleich zu Wasser möglich. Durch diese Verkürzung macht sich der eingangs genannte Effekt, nämlich ein Abfall des Druckes am Ort der Linse zum Rand hin, nicht mehr so ausgeprägt bemerkbar wie bei einer längeren Vorlaufstrecke mit Wasser. Die Apertur der Fokussierungseinrichtung wird wesentlich besser ausgenutzt, was einen günstigeren Öffnungswinkel ergibt, und es ergibt sich ein Längen/Breiten-Verhältnis der Fokuszone, welches günstiger ist als bei einer Füllung der Vorlaufstrecke mit Wasser.

Als weitere Stoffe für die Vorlaufstrecke kommen beispielsweise einwertige Alkohole, wie etwa Äthanol mit einem B/A-Wert von 11 und Benzol in Betracht.

Weitere Vorteile und Ausgestaltungen der Erfindung ergeben sich aus der Beschreibung eines Ausführungsbeispiels anhand der Figuren. Es zeigen:

Fig. 1 eine Stoßwellenquelle mit Druckquelle und Fokussierungseinrichtung gemäß dem Stand der Technik und

Fig. 2 eine Stoßwellenquelle mit Druckquelle und Fokussierungseinrichtung mit verbesserter Fokuszone.

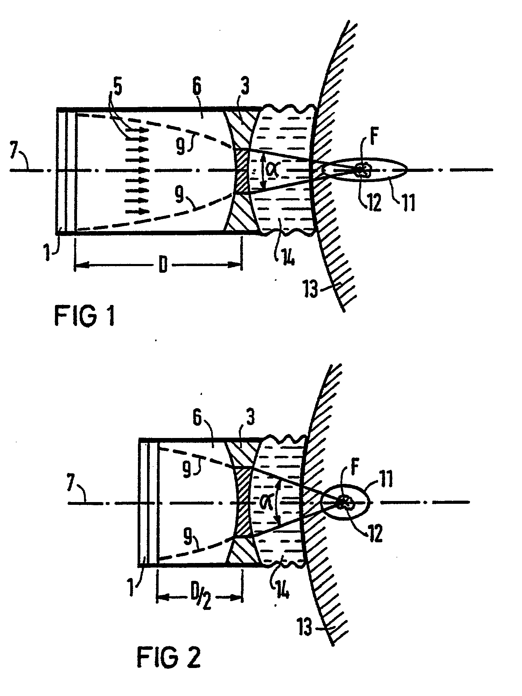

In Fig. 1 ist mit 1 eine Druckquelle bezeichnet, die beispielsweise ein Stoßwellenrohr sein kann, z. B. nach der DE-OS 33 28 051. In einem vorgegebenen Abstand D von der Druckquelle 1 entfernt befindet sich eine Fokussierungseinrichtung 3. Der Raum, den ein Druckwellenimpuls 5 zwischen der Druckquelle 1 und der Fokussierungseinrichtung 3 durchqueren muß, ist üblicherweise mit Wasser gefüllt. Er wird als Vorlaufstrecke 6 bezeichnet. Die Druckquelle 1 und die Fokussierungseinrichtung 3 weisen eine gemeinsame Zentrumsachse 7 auf.

In einem durch die Krümmung der Linse, die als Fokussierungseinrichtung 3 dient, vorgegebenen Abstand befindet sich deren geometrischer Fokus F. Am Ort der Fokussierungseinrichtung 3 muß der Quotient aus Amplitude und Anstiegszeit des Druckwellenimpulses 5 einen Mindestwert haben, damit sich im weiteren Verlauf bis zum Fokus F eine Stoßwelle ausbilden kann. Der Druckwellenimpuls 5 steilt sich beim Lauf durch die Vorlaufstrecke 6 soweit auf, daß der Quotient am Ort der Linse seinen vorgegebenen Wert erreicht. Nachteilig ist dabei, daß die Druckamplitude des Druckwellenimpulses 5 radial nach außen, also zum Rand hin, abfällt. Als Folge davon steht am Ort der Fokussierungseinrichtung 3 ein Druckwellenimpuls 5 ' zur Verfügung, der nur im mittleren Bereich ein schmales Maximum aufweist. Die beiden mit 9 bezeichneten Linien geben die -6dB-Isobare der Druckamplitude bezogen auf das zentrale Druckmaximum an. Es wird effektiv nur noch ein Teil der Apertur der Fokussierungseinrichtung 3 ausgenutzt. Die am Rand der Fokussierungseinrichtung 3 verlaufenden Teile des Druckwellenimpulses 5′ tragen wenig zur späteren Bildung der Stoßwelle bei.

Die -6dB-Isobare in der Fokuszone 11 um den Fokus F herum weist aufgrund der kleinen effektiven Apertur ein Längen/Breiten-Verhältnis von ca. 8 auf. Im Fokus F ist, wie üblich, das zu zerstörende Konkrement 12 eines menschlichen Patienten 13 plaziert. Zwischen der Fokussierungseinrichtung 3 und dem Patienten 13 liegt eine mit Flüssigkeit gefüllte Koppelstrecke 14. Es ist wünschenswert, das Längen/Breiten-Verhältnis der Fokuszone 11 zu verbessern. Außerdem ist es wünschenswert, einen größeren Öffnungswinkel α zum Einstrahlen des Druckwellenimpulses 5 in den Körper des Patienten 13 zu erzielen.

Als Nachteil ist weiter zu verzeichnen, daß aufgrund des nicht voll ausgenutzten wirksamen Querschnitts der Fokussierungseinrichtung 3 die Amplitude des anfänglichen Druckwellenimpulses 5′ größer sein muß als wenn die volle Apertur zur Bildung der späteren Stoßwelle beitragen würde. Die genannten Eigenschaften lassen sich mit der in Fig. 2 gezeigten Ausführungsform eines Lithotripters verbessern.

In Fig. 2 sind gleiche Teile mit gleichen Bezugszeichen versehen wie in Fig. 1. Im Gegensatz zu Fig. 1 ist die Vorlaufstrecke 6 zwischen der Druckquelle 1 und der Fokussierungseinrichtung 3 nicht mit Wasser, sondern mit einem flüssigen Stoff mit einem hohen B/A-Verhältnis und einer akustischen Impedanz gefüllt, die höchstens so groß ist, wie die von Wasser. Eine solche Substanz ist vorzugsweise Rizinusöl. Auch mit einem einwertigen Alkohol, wie Äthanol oder mit Benzol, wird eine zufriedenstellende Verbesserung der zuvor beschriebenen Effekte erzielt.

Aufgrund des Stoffes mit dem hohen B/A-Verhältnis bei gleichzeitig (im Vergleich zu der des Wassers) kleinerer oder gleich großer akustischer Impedanz steilt sich der Druckwellenimpuls 5 in der Vorlaufstrecke sehr viel schneller auf. Dadurch wird der durch konstruktive Gegebenenheiten vorgegebene Mindestwert des Quotienten von Amplitude zu Anstiegszeit am Ort des Druckwellenimpulses 5 am Ort der Fokussierungseinrichtung 3 früher erreicht, als bei einer Füllung mit Wasser. Das hat zur Folge, daß die Druckquelle 1 nicht mehr den gesamten Abstand D zur Fokussierungseinrichtung 3 einnehmen muß, sondern daß der Abstand D größenordnungsmäßig auf die Hälfte reduziert werden kann. Dies ist in Fig. 2 gezeigt. Durch den kürzeren Abstand von z. B. D/2 weisen die Linien 9, für die -6dB-Isobaren, am Ort der Fokussierungseinrichtung 3 einen größeren Abstand voneinander auf. Die wirksame Apertur der Linse ist gegenüber der Darstellung von Fig. 1 mit Wasserfüllung in der Vorlaufstrecke 6 wesentlich vergrößert. Dadurch ergibt sich zum einen ein größerer effektiver Einstrahlwinkel α und ein günstigeres Längen-Breiten-Verhältnis der Fokuszone 11. Als weiterer Vorteil ergibt sich eine kürzere Bauweise der Anordnung und ein besserer Wirkungsgrad. Der bessere Wirkungsgrad kommt zum einen durch die günstigere Verteilung des Druckwellenimpulses 5 auf die wirksame Apertur der Fokussierungseinrichtung 3 und zum anderen durch kleinere Ultraschallverluste in der Vorlaufstrecke 6 zustande.

Die genannten Vorteile ergeben sich im gleichen Maße, wenn als Fokussierungseinrichtung 3 ein Reflektor eingesetzt wird. Ebenso muß die Druckquelle 1 nicht notwendigerweise einen ebenen Druckwellenimpuls 5 erzeugen.

Das schnelle Aufsteilen des Druckwellenimpulses 5 wird bei Betrachtung der Formel

$$s \propto \frac{\rho \, c^3 \, \Delta t}{p(2+B/A)}$$

verständlich. Darin bedeuten S = Strecke bis zur Bildung einer Stoßwelle; p = Amplitude, $\Delta$ t = Anstiegszeit des Druckwellenimpulses 5; $\rho$ = Dichte des Stoffes in der Vorlaufstrecke; c = Schallgeschwindigkeit des Stoffes; B/A = Parameter zur Beschreibung des nichtlinearen Verhaltens des Stoffes wie aus der Literatur bekannt.

Für verschiedene, mögliche Stoffe ergeben sich die folgenden Werte für

$$\rho, \ c, \ \rho c, \ B/A, \ \frac{\rho c^3}{2+B/A} :$$

| | $\rho$ | $c$ | $\rho c$ | $B/A$ | $\frac{\rho^3 c}{2+B/A}$ |
|---|---|---|---|---|---|
| | $10^3 \frac{kg}{m^3}$ | $10^3 \frac{m}{s}$ | $10^6 \frac{kg}{m^2 s}$ | | $10^{11} \frac{kg}{s^3}$ |
| Wasser | 1 | 1,48 | 1,48 | 5,1 | 4,6 |
| Rizinusöl | 0,95 | 1,54 | 1,45 | 10 | 2,9 |
| Äthanol | 0,79 | 1,18 | 0,93 | 11 | 1 |

Die sich ergebende Wirkung ist so zu erklären, daß der Energieverlust des Druckwellenimpulses 5 während der Ausbreitung größer ist als die Verluste durch Dämpfung der niederfrequenten Anteile nahe an der Druckquelle 1. In größerem Abstand von der Druckquelle 1 kehrt sich mit der Zunahme höherfrequenter Anteile die Situation um. Der Einfluß der Anstiegszeit zeigt sich darin, daß bei einer Druckquelle 1, bei der die Entladung sehr schnell erfolgt, die Strecke 6, in der das Einbringen eines Rizinusöls noch einen positiven Effekt bringt, wesentlich verkürzt ist.

Eine andere Verwendung der beschriebenen Stoßwellenquelle ist die Behandlung im Körper oder auf der Hand liegender Tumore mit Stoßwellen.

## Patentansprüche

1. Stoßwellenquelle, insbesondere für einen Lithotripter, mit einer Druckquelle zur Erzeugung eines Druckwellenimpulses und mit einer im Ausbreitungsweg des Druckwellenimpulses und im Abstand von der Druckquelle angeordneten Fokussierungseinrichtung zur Fokussierung des Druckwellenimpulses, dadurch gekennzeichnet, daß sich zwischen der Druckquelle (1) und der Fokussierungseinrichtung (3) ein Stoff (6) befindet, dessen B/A-Wert größer als der von Wasser und dessen akustische Impedanz kleiner oder gleich der von Wasser ist.

2. Stoßwellenquelle nach Anspruch 1, dadurch gekennzeichnet, daß der B/A-Wert des zwischen der Druckquelle (1) und der Fokussierungseinrichtung (3) befindlichen Stoffes 10 oder 11 beträgt.

3. Stoßwellenquelle nach Anspruch 1 oder 2, dadurch gekennzeichnet, daß der Stoff Rizinusöl ist.

4. Stoßwellenquelle nach Anspruch 1 oder 2, dadurch gekennzeichnet, daß der Stoff ein einwertiger Alkohol ist.

5. Stoßwellenquelle nach Anspruch 4, dadurch gekennzeichnet, daß der Stoff Äthanol ist.

6. Stoßwellenquelle nach Anspruch 1 oder 2, dadurch gekennzeichnet, daß der Stoff Benzol ist.

## Claims

1. Shock wave source, in particular for a lithotriptor, having a pressure source for producing a pressure wave pulse and a focussing device for focussing the pressure wave pulse arranged in the propagation path of the pressure wave pulse and at a distance from the pressure source, characterised in that a substance (6), with a B/A value greater than that of water and an acoustic impedance less than or the same as that of water, is arranged between the pressure source (1) and the focussing device (3).

2. Shock wave source according to claim 1, characterised in that the B/A value of the substance between the pressure source (1) and the focussing device (3) is 10 or 11.

3. Shock wave source according to claim 1 or 2, characterised in that the substance is castor oil.

4. Shock wave source according to claim 1 or 2, characterised in that the substance is a monohydric alcohol.

5. Shock wave source according to claim 4, characterised in that the substance is ethanol.

6. Shock wave source according to claim 1 or 2, characterised in that the substance is benzene.

**Revendications**

1. Générateur d'ondes de choc, notamment pour un lithotriteur, comportant une source de pression servant à produire une impulsion d'onde de pression et un dispositif de focalisation disposé dans le trajet de propagation de l'impulsion d'onde de pression et à distance de la source de pression et servant à focaliser l'impulsion d'onde de pression, caractérisé par le fait qu'entre la source de pression (1) et le dispositif de focalisation (3) est placée une substance (6), dont la valeur B/A est supérieure à celle de l'eau et dont l'impédance acoustique est inférieure ou égale à celle de l'eau.

2. Générateur d'ondes de choc selon la revendication 1, caractérisée par le fait que la valeur B/A de la substance située entre la source de pression (1) et le dispositif de focalisation (3) est égale à 10 ou 11.

3. Générateur d'ondes de choc suivant la revendication 1 ou 2, caractérisé par le fait que la substance est de l'huile de ricin.

4. Générateur d'ondes de choc suivant la revendication 1 ou 2, caractérisé par le fait que la substance est un alcool monovalent.

5. Générateur d'ondes de choc suivant la revendication 4, caractérisé par le fait que la substance est de l'éthanol.

6. Générateur d'ondes de choc suivant la revendication 1 ou 2, caractérisé par le fait que la substance est du benzène.

FIG 1

FIG 2